(19) Europäisches Patentamt / European Patent Office / Office européen des brevets

(11) **EP 4 073 473 B1**

(12) **EUROPÄISCHE PATENTSCHRIFT**

(45) Veröffentlichungstag und Bekanntmachung des Hinweises auf die Patenterteilung:
**04.09.2024 Patentblatt 2024/36**

(21) Anmeldenummer: **20811586.5**

(22) Anmeldetag: **20.11.2020**

(51) Internationale Patentklassifikation (IPC):
*G01D 21/00* (2006.01) *H03M 1/00* (2006.01)

(52) Gemeinsame Patentklassifikation (CPC):
**G01D 21/00**

(86) Internationale Anmeldenummer:
**PCT/EP2020/082901**

(87) Internationale Veröffentlichungsnummer:
**WO 2021/115760 (17.06.2021 Gazette 2021/24)**

(54) **VERFAHREN ZUR OPTIMIERUNG EINER MESS-RATE EINES FELDGERÄTES**

METHOD FOR OPTIMIZING A MEASUREMENT RATE OF A FIELD DEVICE

PROCÉDÉ D'OPTIMISATION D'UN DÉBIT DE MESURE D'UN DISPOSITIF DE TERRAIN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.12.2019 DE 102019133600**

(43) Veröffentlichungstag der Anmeldung:
**19.10.2022 Patentblatt 2022/42**

(73) Patentinhaber: **Endress+Hauser SE+Co. KG 79689 Maulburg (DE)**

(72) Erfinder:
• FRÜHAUF, Dietmar
  79539 Lörrach (DE)
• BRAUN, Marco
  85737 Ismaning (DE)
• FRANKE, Alexander
  4127 Birsfelden (CH)

(74) Vertreter: **Koslowski, Christine Adelheid Endress+Hauser Group Services (Deutschland) AG+Co. KG Colmarer Straße 6 79576 Weil am Rhein (DE)**

(56) Entgegenhaltungen:
DE-A1- 102008 016 940    US-A1- 2018 019 910
US-A1- 2019 278 236

• TAYEH GABY BOU ET AL: "A Spatial-Temporal Correlation Approach for Data Reduction in Cluster-Based Sensor Networks", IEEE ACCESS, vol. 7, 26 April 2019 (2019-04-26), pages 50669 - 50680, XP011721293, DOI: 10.1109/ACCESS.2019.2910886
• GHAHRAMANI Z ET AL: "Supervised Learning from Incomplete Data via an EM Approach", ADVANCES IN NEURAL INFORMATION PROCESSING SYSTEMS, XX, XX, vol. 6, 29 November 1993 (1993-11-29), pages 120 - 127, XP007904552

**Beschreibung**

Die Erfindung betrifft ein Verfahren zur Optimierung einer Mess-Rate eines Feldgerätes in einem Mess-System.

In der Automatisierungstechnik, insbesondere zur Prozessautomatisierung, werden vielfach Feldgeräte eingesetzt, die zur Erfassung verschiedener Messgrößen dienen. Bei der zu bestimmenden Messgröße kann es sich beispielsweise um einen Füllstand, einen Durchfluss, einen Druck, die Temperatur, den pH-Wert, das Redoxpotential, eine Leitfähigkeit oder den Dielektrizitätswert eines Mediums in einer Prozessanlage handeln. Zur Erfassung der entsprechenden Messwerte umfassen die Feldgeräte jeweils geeignete Sensoren bzw. basieren auf geeigneten Messprinzipien. Eine Vielzahl verschiedener Feldgeräte-Typen wird von der Firmen-Gruppe Endress + Hauser hergestellt und vertrieben.

In der jeweiligen Prozessanlage sind die einzelnen Feldgeräte in der Regel zu einem Mess-System zusammengeschaltet, um die korrespondierenden Prozessvariablen mit geeigneten Aktoren, wie Heitz-Elementen, Rührwerken, Ventilen oder Pumpen für Zu- und Abläufe koordinieren zu können. Dementsprechend korrelieren auch die Messgrößen der einzelnen Feldgeräte einer Prozessanlage gegebenenfalls miteinander. Zur Kommunikation innerhalb des Mess-Systems können die Feldgeräte entweder direkt, oder auch zentral über eine Steuer-Einheit, wie eine Prozessleitstelle, miteinander verbunden sein. Vor allem bei Feldgeräten, die an schwer zugänglichen Orten der Prozessanlage angeordnet sind, werden zur Kommunikation innerhalb des Mess-Systems vorzugsweise drahtlose Übertragungsprotokolle, wie Wireless HART oder WLAN implementiert. Die Energieversorgung dieser Feldgeräte erfolgt in diesen Fällen entsprechend über Batterien. Die potentielle Einsatzdauer des einzelnen Feldgerätes hängt dabei von der Kapazität der Batterie sowie der Mess-Rate, also der Taktung und der Messzeit pro Takt, mit der das Feldgerät den Messwert misst, ab.

In diesem Zusammenhang darf die Mess-Rate jedoch auch nicht zu gering eingestellt werden, damit der Prozess ausreichend überwacht werden kann. In Verbindung mit der endlichen Kapazität der Batterie müssen die entsprechenden Feldgeräte daher in regelmäßigem Wartungszyklen außer Betrieb genommen werden, um den Batterie-Wechsel vorzunehmen. Hierdurch bedingt müssen auch die Prozesse innerhalb der Prozessanlage in diesen Wartungszeiträumen gestoppt werden, da die Prozesse ohne entsprechende Überwachung in der Regel nicht kontrolliert ablaufen können. Dies ist insofern nachteilhaft für den Anlagenbetreiber, da jegliche Stillstandszeit der Prozessanlage dessen Wirtschaftlichkeit beeinträchtigt.

TAYEH GABY BOU ET AL: "A Spatial-Temporal Correlation Approach for Data Reduction in Cluster-Based Sensor Networks" offenbart ein Verfahren zur Datenreduktion mittels räumlicher und zeitlicher Korrelation von Sensormessdaten.

Der Erfindung liegt daher die Aufgabe zugrunde, ein Verfahren bereitzustellen, mit dem die Verfügbarkeit von Batteriebetriebenen Feldgeräten erhöht werden kann.

Die Erfindung löst diese Aufgabe durch ein Verfahren zur Optimierung einer Mess-Rate eines ersten Feldgerätes in einem Mess-System. Zur Anwendung des Verfahrens ist es hierbei erforderlich, dass das Mess-System neben dem ersten Feldgerät zumindest ein zweites Feldgerät umfasst, wobei die zumindest zwei Feldgeräte mit jeweils einer bestimmten Mess-Rate Messwerte entsprechender Messgrößen messen, und wobei zumindest die Messgröße des ersten Feldgerätes mit der Messgröße des zweiten Feldgerätes korreliert. Folgende Verfahrensschritte umfasst das Verfahren:

- Messung der Messgrößen der zumindest zwei Feldgeräte mit jeweils einer voreingestellten Mess-Rate während einer definierten Einlernphase,
- Bestimmung eines Korrelations-Musters zwischen der ersten Messgröße und der zweiten Messgröße anhand der in der Einlernphase gemessenen Messwerte.

Dabei werden zumindest die Messwerte des zweiten Feldgerätes während eines Messbetriebs auf das Korrelations-Muster hin geprüft, wobei die Mess-Rate des ersten Feldgerätes im Messbetrieb zumindest während desjenigen Zeitfensters, in dem das Korrelations-Muster bei den Messwerten der zweiten Messgröße erkannt wird, geändert wird.

Dabei kann das optimale Korrelations-Muster, wie beispielsweise eine Pearson- oder Partial- Korrelation mittels eines Auto-Machine-Learning Algorithmus bestimmt werden.

Die Erfindung beruht also darauf, die Korrelation der einzelnen Messgrößen zueinander zu überprüfen. Wird durch das Verfahren eine starke Korrelation zwischen den Messgrößen erkannt, so wird dies als Beleg für die Funktionsfähigkeit des Mess-Systems gewertet. Im anderen Fall ist von einer Störung des Mess-Systems auszugehen, so dass die gemessenen Messwerte als falsch bzw. zumindest nicht vertrauenswürdig einzustufen sind.

Die Mess-Rate des ersten Feldgerätes kann im Messbetrieb zumindest während des Zeitfensters, in dem das Korrelations-Muster bei den Messwerten der zweiten Messgröße erkannt wird, verringert werden. Hierdurch kann der Leistungsverbrauch des jeweiligen Gerätes verringert werden. Vorteilhaft ist dies vor allem, wenn das erste Feldgerät und/oder das zweite Feldgerät zur Energieversorgung eine Batterie umfassen, da hierdurch die Batterie-Laufzeit und somit die Verfügbarkeit in der Prozessanlage erhöht werden.

Um etwaigen Rechenaufwand zu vermindern und somit die Erstellung des Korrelations-Musters zu beschleunigen, können zur Bestimmung des Korrelations-Musters redundante Messwerte aus der Einlernphase vorteilhafter Weise beispielsweise mittels eines Unsupervised Clustering Verfahrens herausgefiltert werden.

Ein entsprechendes Mess-System, das zur Ausführung des Verfahrens gemäß einer der vorhergehenden Ausführungsvarianten geeignet ist, hat zumindest folgende Komponenten zu umfassen:

- Eine erstes Feldgerät, das ausgelegt ist, die erste Messgröße mit einer einstellbaren Mess-Rate zu messen,
- eine zweites Feldgerät, das ausgelegt ist, die zweite Messgröße zu messen,
- eine Steuer-Einheit, die ausgelegt ist, um

  ◦ ein Korrelations-Muster zwischen der ersten Messgröße und der zweiten Messgröße anhand der in der Einlernphase gemessenen Messwerte zu bestimmen,
  ◦ zumindest die Messwerte des zweiten Feldgerätes während des Messbetriebs auf das Korrelations-Muster hin zu prüfen,
  ◦ zumindest die Mess-Rate des ersten Feldgerätes im Messbetrieb zumindest während eines Zeitfensters, in dem das Korrelations-Muster bei den Messwerten der zweiten Messgröße erkannt wird, zu ändern.

Dabei können das erste Feldgerät und/oder das zweite Feldgerät beispielsweise mittels einer drahtlosen Schnittstelle mit der Steuer-Einheit verbunden sein.

Unter dem Begriff "*Einheit*" werden im Rahmen der Erfindung prinzipiell jede elektronische Schaltung verstanden, die für den angedachten Einsatzzweck geeignet ausgelegt ist. Es kann sich also je nach Anforderung um eine Analogschaltung zur Erzeugung bzw. Verarbeitung entsprechender analoger Signale handeln. Es kann sich jedoch auch um eine Digitalschaltung wie einem FPGA oder einen Speichermedium in Zusammenwirken mit einem Programm handeln. Dabei ist das Programm ausgelegt, die entsprechenden Verfahrensschritte durchzuführen bzw. die notwendigen Rechenoperationen der jeweiligen Einheit anzuwenden. In diesem Kontext können verschiedene elektronische Einheiten des Füllstandsmessgerätes im Sinne der Erfindung potentiell auch auf einen gemeinsamen physikalischen Speicher zurückgreifen bzw. mittels derselben physikalischen Digitalschaltung betrieben werden.

Anhand der nachfolgenden Figuren wird die Erfindung näher erläutert. Es zeigt:

Fig. 1: Ein Mess-System mit drei Feldgeräten in einer Prozessanlage, und

Fig. 2: eine Korrelation zwischen den Messgrößen der Feldgeräte.

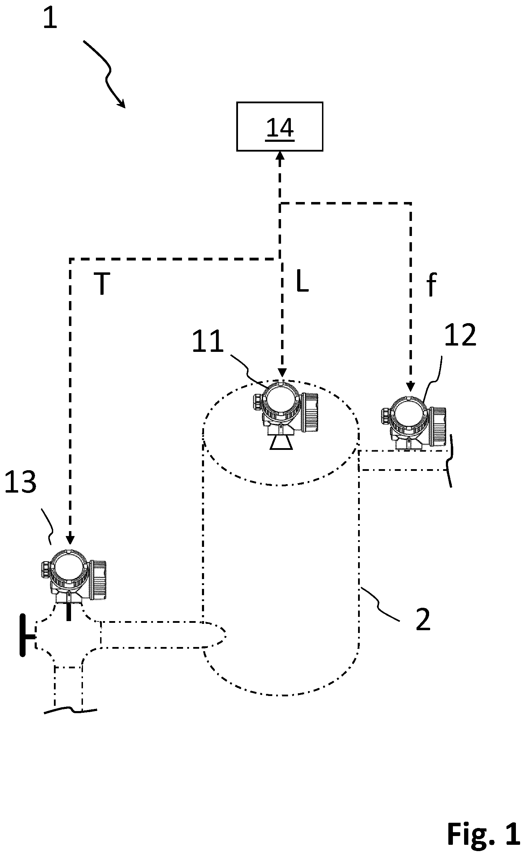

Zum allgemeinen Verständnis des erfindungsgemäßen Verfahrens ist in Fig. 1 ein beispielhaftes Mess-System 1 gezeigt, das zur Überwachung einer Prozess-Anlage 2, wie beispielsweise einem Chemie-Reaktor, dient. Hierzu umfasst das exemplarische Mess-System 1 als Feldgeräte ein Durchflussmessgerät 12 an einem Zulauf des Reaktors 2, ein Füllstandsmessgerät 11 am Reaktor 2 selbst, und ein Temperaturmessgerät 13 an einem Ablauf des Reaktors 2. Dabei messen die Feldgeräte 11, 12, 13 die entsprechenden Messwerte L, f, T jeweils mit einer individuell einstellbaren Mess-Rate, beispielsweise zwischen 1 Messung pro Minute und 1000 Messungen pro Sekunde.

Über den Zulauf des Reaktors 2 können beispielsweise Reaktions-Edukte zugeführt werden, wobei die Durchflussrate f, mit der das Reaktions-Edukt zugeführt wird, mittels des Durchflussmessgerätes 12 erfasst wird.

Das Füllstandsmessgerät 11 misst den Füllstand L im Reaktor 2 und überwacht somit beispielsweise, ob durch die Reaktion oder durch das Zuführen des Reaktions-Eduktes ein kritischer Füllstandswert L über- oder unterschritten wird. Dementsprechend korrelieren die Messwerte des Füllstandsmessgerätes 11 in der exemplarischen Prozessanlage 2 insofern mit den Messwerten des Durchflussmessgerätes 12, als dass sich in demjenigen Zeitintervall $\Delta t$, in dem eine konstante Durchflussrate f im Zulauf vorherrscht, sich der Füllstand L im Reaktor 2 zeitlich linear erhöht. Somit kann diese exemplarische Korrelation des Füllstandes L in Bezug zum Durchfluss f funktional beschrieben werden, da der Füllstand durch die Stammfunktion des Durchflusses gebildet wird:

$$L(t) = const. + \int\limits^{\Delta t} f(t)dt$$

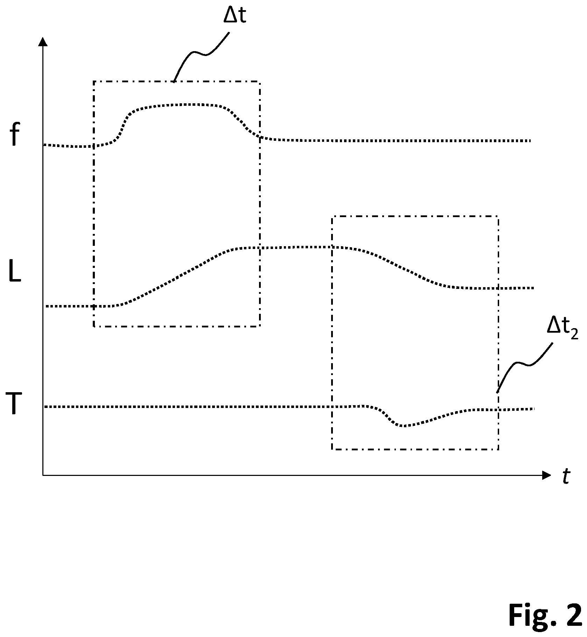

Schematisch dargestellt ist diese Korrelation der Füllstands-Messwerte mit denen des Durchflussmessgerätes 12 in dem Graph von Fig. 2

Das Temperaturmessgerät 13 am Ablauf des Reaktors 2 kann bei dem in Fig. 1 gezeigten Ausführungsbeispiel wiederum dazu dienen, die Temperatur T eines Reaktions-Produktes beim Entleeren des Reaktors 2 zu messen, um beispielsweise nachfolgende Prozess-Schritte entsprechend anzupassen. Sofern etwaige chemische Reaktionen in der Prozessanlage 2 endotherm verlaufen und daher zu einer Abkühlung im Reaktor 2 führen, registriert das Temperaturmessgerät 13 während des entsprechenden Zeitintervalls $\Delta t_2$, in dem der Reaktor 2 entleert wird, je nach Umgebungstemperatur zumindest einen kurzen Abfall der Temperatur T. Dementsprechend korrelieren auch die Messwerte des Temperaturmessgerätes 13 bei einer (zeitlich linearen) Abnahme des Füllstandes L mit den Messwerten des Füllstandsmessgerätes 11. Auch dieser exemplarische Zusammenhang ist schematisch in dem Graph von Fig. 2 dargestellt.

Bei der in Fig. 1 gezeigten Ausführungsvariante umfasst das Mess-System 1 eine Steuer-Einheit 14, mit welcher die Feldgeräte 11, 12, 13 verbunden sind. Dabei kann es sich bei der Steuer-Einheit 14 beispielsweise um das Prozessleitsystem der Prozessanlage handeln. Als Schnittstelle, über welche die Feldgeräte 11, 12, 13 mit der Steuer-Einheit 14 verbunden sind, kann etwa "PROFIBUS", "HART", "Wireless HART" oder "Ethernet" implementiert sein. Speziell bei kabelloser Auslegung der Schnittstellen können die Feldgeräte 11, 12, 13 entsprechend mittels Batterie betrieben werden, so dass hierfür keine zusätzliche Verkabelung erforderlich ist.

Über die Schnittstellen können die von den Feldgeräten 11, 12, 13 gemessenen Messwerte f, L, T übermittelt werden. Bei entsprechender Auslegung ist es der Steuer-Einheit 4 hierdurch möglich, während einer definierten Einlernphase die zuvor beschriebenen Korrelations-Muster zwischen den Füllstands-Messwerten und den Messwerten f des Durchflussmessgerätes 12 bzw. den Temperaturmesswerten und den Füllstands-Messwerten zu bestimmen. Zur Findung eines geeigneten Korrelations-Typs, wie beispielsweise einer Pearson- oder Partial-Korrelation kann die Steuer-Einheit 14 beispielsweise einen Auto-Machine-Learning Algorithmus anwenden.

Nach Erfassung der Korrelations-Muster kann das erfindungsgemäße Mess-System 1 bzw. die Steuer-Einheit 14 in den regulären Messbetrieb übergehen. Das heißt, während des Messbetriebs prüft die die Steuer-Einheit 14 zumindest die Messwerte f des Durchflussmessgerätes 12 auf das zuvor bestimmte Korrelations-Muster hin. Konkret wird geprüft, ob eine derzeit eine (konstanter) Durchfluss-Rate f vorherrscht. Sofern, dies erkannt wird, wird hieraus abgeleitet, dass sich aufgrund des zuvor erkannten Korrelations-Musters auch der Füllstand L entsprechend ändern muss.

Da aufgrund dieser Art der Korrelation die Füllstands-Änderung vorhersehbar ist, kann infolge dessen die Mess-Rate des Füllstandsmessgerätes 11 im Messbetrieb zumindest während desjenigen Zeitfensters Δt, in dem das Korrelations-Muster bei den Durchfluss-Messwerten f erkannt wird, verringert werden, ohne das bezüglich des Füllstandes L eine unvorhersehbare abrupte Füllstands-Änderung zu erwarten ist. Hierdurch kann für den Fall, dass das Füllstandsmessgerät 11 Batterie-betrieben ist, dessen Einsatzdauer und somit dessen Verfügbarkeit optimiert werden.

Analog zu den Durchfluss-Messwerten f des Durchflussmessgerätes 12 kann die Steuer-Einheit 14 während des Messbetriebes auch die Messwerte L des Füllstandsmessgerätes 11 auf das zuvor festgelegte Korrelations-Muster des Temperaturmessgerätes 13 hinsichtlich der Füllstands-Messwerte L hin überprüfen. Sobald also eine Füllstands-Abnahme detektiert wird, wird dies wiederum als Vorliegen des Korrelations-Musters erkannt und eine entsprechende (kurzzeitige) Senkung der Temperatur T am TemperaturMessgerät 13 antizipiert. Folglich kann auch die Mess-Rate des Temperatur-Messgerätes 13 im Messbetrieb zumindest während des Zeitfensters Δt$_2$, in dem das Korrelations-Muster bei den Füllstands-Werten L erkannt wird, verringert werden, ohne dass eine unvorhergesehene Temperatur-Änderung zu erwarten ist. Somit können also auch die Einsatzdauer bzw. die Verfügbarkeit des Temperatur-Messgerätes 13 im Falle von Batterie-Betrieb erhöht werden.

Die Steuer-Einheit 14 ist in der gezeigten Darstellung als separate, übergeordnete Einheit dargestellt. Im Rahmen der Erfindung ist es jedoch ebenso denkbar, die Steuer-Einheit 4 nicht als externes Gerät, sondern als Bestandteil eines der Feldgeräte 11, 12, 13 auszulegen.

**Bezugszeichenliste**

| | |
|---|---|
| 1 | Mess-System |
| 2 | Prozess-Anlage |
| 11 | Füllstandsmessgerät |
| 12 | Durchflussmessgerät |
| 13 | Temperaturmessgerät |
| 14 | Steuer-Einheit |
| f | Durchfluss-Rate |
| L | Füllstand |
| T | Temperatur |
| Δt | Zeitfenster |

**Patentansprüche**

1. Verfahren zur Optimierung einer Mess-Rate eines ersten Feldgerätes (11) in einem Mess-System (1), wobei das erste Feldgerät (11) ausgelegt ist, eine erste Messgröße (L) mit einer einstellbaren Mess-Rate zu messen, wobei das Mess-System (1) neben dem ersten Feldgerät (11) zumindest ein zweites Feldgerät (12) umfasst, welches ausgelegt ist, eine zweiten Messgröße (f) zu messen, wobei die erste Messgröße (L) von der zweiten Messgröße (f) verschieden ist und wobei zumindest die erste Messgröße (L) des ersten Feldgerätes (11) mit der zweiten Messgröße (f) des zweiten Feldgerätes (12) korreliert, folgende Verfahrensschritte umfassend:

- Messung der Messgrößen (f, L) der zumindest zwei Feldgeräte (11, 12) mit jeweils einer voreingestellten Mess-Rate während einer definierten Einlernphase,
- Bestimmung eines Korrelations-Musters zwischen der ersten Messgröße (L) und der zweiten Messgröße (f) anhand der in der Einlernphase gemessenen Messwerte,
- Durchführung eines Messbetriebs,
wobei zumindest die Messwerte des zweiten Feldgerätes (12) während des Messbetriebs auf das Korrelations-Muster hin geprüft werden, und

wobei die Mess-Rate des ersten Feldgerätes (11) im Messbetrieb zumindest während eines Zeitfensters (Δt), in dem das Korrelations-Muster bei den Messwerten der zweiten Messgröße (f) erkannt wird, geändert wird.

2. Verfahren nach Anspruch 1, wobei das Korrelations-Muster mittels eines Auto-Machine-Learning Algorithmus bestimmt wird.

3. Verfahren nach einem der vorhergehenden Ansprüche, wobei die Mess-Rate des ersten Feldgerätes (11) im Messbetrieb zumindest während des Zeitfensters (Δt), in dem das Korrelations-Muster bei den Messwerten der zweiten Messgröße (f) erkannt wird, verringert wird.

4. Verfahren nach Anspruch 1 bis 3, wobei zur Bestimmung des Korrelations-Musters redundante Messwerte aus der Einlernphase insbesondere mittels eines Unsupervised Clustering Verfahrens herausgefiltert werden.

5. Mess-System zur Ausführung des Verfahrens nach einem der vorherigen Ansprüche, umfassend:

   - Ein erstes Feldgerät (11), das ausgelegt ist, die erste Messgröße (L) mit einer einstellbaren Mess-Rate zu messen,
   - eine zweites Feldgerät (12), das ausgelegt ist, die zweite Messgröße (f) zu messen, wobei die erste Messgröße (L) von der zweiten Messgröße (f) verschieden ist,
   - eine Steuer-Einheit (4), die ausgelegt ist, um

     ◦ ein Korrelations-Muster zwischen der ersten Messgröße (L) und der zweiten Messgröße (f) anhand der in der Einlernphase gemessenen Messwerte zu bestimmen,
     ◦ zumindest die Messwerte des zweiten Feldgerätes (12) während des Messbetriebs auf das Korrelations-Muster hin zu prüfen,
     ◦ zumindest die Mess-Rate des ersten Feldgerätes (11) im Messbetrieb zumindest während eines Zeitfensters (Δt, Δt$_2$), in dem das Korrelations-Muster bei den Messwerten der zweiten Messgröße (f) erkannt wird, zu ändern.

6. Mess-System nach Anspruch 5, wobei das erste Feldgerät (11) und/oder das zweite Feldgerät (12) zur Energieversorgung eine Batterie umfassen bzw. umfasst.

7. Mess-System nach Anspruch 5 oder 6, wobei das erste Feldgerät (11) und/oder das zweite Feldgerät (12) mittels einer drahtlosen Schnittstelle mit der Steuer-Einheit (14) verbunden sind bzw. ist.

**Claims**

1. A method for optimizing a measurement rate of a first field device (11) in a measuring system (1), wherein the first field device (11) is configured to measure a first measured variable (L) with an adjustable measurement rate, wherein the measuring system (1), in addition to the first field device (11), comprises at least a second field device (12), which is configured to measure a second measured variable (f), wherein the first measured variable (L) is different from the second measured variable (f), and wherein at least the first measured variable (L) of the first field device (11) correlates with the second measured variable (f) of the second field device (12), comprising the following process steps:

   - Measuring the measured variables (f, L) of the at least two field devices (11, 12) each with a preset measurement rate during a defined calibration phrase,
   - working out a correlation pattern between the first measured variable (L) and the second measured variable (f) based on the measured values from the calibration phase,
   - implementing measuring mode,
   wherein at least the measured values of the second field device (12) are checked in relation to the correlation pattern during measuring mode, and
   wherein the measurement rate of the first field device (11) in measuring mode is changed at least during a time window (Δt) in which the correlation pattern is detected in the measured values of the second measured variable (f).

2. The method as claimed in claim 1, wherein the correlation pattern is worked out using an automated machine learning algorithm.

3. The method as claimed in one of the preceding claims, wherein the measurement rate of the first field device (11) in measuring mode is reduced at least during the time window (Δt) in which the correlation pattern is detected in the measured values of the second measured variable (f).

4. The method as claimed in claims 1 to 3, wherein redundant measured values from the calibration phase are filtered out, in particular using an unsupervised clustering method, in order to work out the correlation pattern.

**5.** A measuring system for implementing the method as claimed in one of the preceding claims, comprising:

- A first field device (11), which is configured to measure the first measured variable (L) with an adjustable measurement rate,
- a second field device (12), which is configured to measure the second measured variable (f), wherein the first measured variable (L) is different from the second measured variable (f),
- a control unit (4), which is configured

  ◦ to work out a correlation pattern between the first measured variable (L) and the second measured variable (f) based on the measured values from the calibration phase,
  ◦ to check at least the measured values of the second field device (12) in relation to the correlation pattern during measuring mode,
  ◦ to change at least the measurement rate of the first field device (11) in measuring mode at least during a time window ($\Delta t$, $\Delta t_2$) in which the correlation pattern is detected in the measured values of the second measured variable (f).

**6.** The measuring system as claimed in claim 5, wherein the first field device (11) and/or the second field device (12) comprise/comprises a battery to supply power.

**7.** The measuring system as claimed in claim 5 or 6, wherein the first field device (11) and/or the second field device (12) are/is connected to the control unit (14) by means of a wireless interface.

**Revendications**

**1.** Procédé destiné à l'optimisation d'une cadence de mesure d'un premier appareil de terrain (11) dans un système de mesure (1), le premier appareil de terrain (11) étant conçu pour mesurer une première grandeur de mesure (L) avec une cadence de mesure réglable, le système de mesure (1) comprenant, outre le premier appareil de terrain (11), au moins un deuxième appareil de terrain (12), lequel est conçu pour mesurer une deuxième grandeur de mesure (f), la première grandeur de mesure (L) étant différente de la deuxième grandeur de mesure (f) et au moins la première grandeur de mesure (L) du premier appareil de terrain (11) étant corrélée avec la deuxième grandeur de mesure (f) du deuxième appareil de terrain (12), lequel procédé comprend les étapes suivantes :

- Mesure des grandeurs de mesure (f, L) des au moins deux appareils de terrain (11, 12) avec respectivement une cadence de mesure préréglée pendant une phase d'apprentissage définie,
- Détermination d'un modèle de corrélation entre la première grandeur de mesure (L) et la deuxième grandeur de mesure (f) à l'aide des valeurs mesurées pendant la phase d'apprentissage,
- Réalisation d'une opération de mesure,

au moins les valeurs mesurées du deuxième appareil de terrain (12) étant contrôlées pendant l'opération de mesure en fonction du modèle de corrélation, et la cadence de mesure du premier appareil de terrain (11) étant modifiée dans l'opération de mesure au moins pendant une fenêtre de temps ($\Delta t$) dans laquelle le modèle de corrélation est identifié pour les valeurs mesurées de la deuxième grandeur de mesure (f).

**2.** Procédé selon la revendication 1, pour lequel le modèle de corrélation est déterminé au moyen d'un algorithme d'Auto-Machine Learning.

**3.** Procédé selon l'une des revendications précédentes, pour lequel la cadence de mesure du premier appareil de terrain (11) est réduite en mode de mesure au moins pendant la fenêtre de temps ($\Delta t$) dans laquelle le modèle de corrélation est identifié pour les valeurs mesurées de la deuxième grandeur de mesure (f).

**4.** Procédé selon les revendications 1 à 3, pour lequel, pour déterminer le modèle de corrélation, des valeurs mesurées redondantes sont filtrées à partir de la phase d'apprentissage, notamment au moyen d'un procédé de clustering non supervisé.

**5.** Système de mesure destiné à la mise en oeuvre du procédé selon l'une des revendications précédentes, lequel système comprend :

- un premier appareil de terrain (11) conçu pour mesurer la première grandeur de mesure (L) avec une cadence de mesure réglable,
- un deuxième appareil de terrain (12) conçu pour mesurer la deuxième grandeur de mesure (f), la première grandeur de mesure (L) étant différente de la deuxième grandeur de mesure (f),
- une unité de commande (4), laquelle est conçue pour

  ◦ déterminer un modèle de corrélation entre la première grandeur de mesure (L) et la deuxième grandeur de mesure (f) à l'aide des valeurs mesurées pendant la phase d'apprentissage,

◦ contrôler au moins les valeurs mesurées du deuxième appareil de terrain (12) pendant le mode de mesure en ce qui concerne le modèle de corrélation,

◦ modifier au moins la cadence de mesure du premier appareil de terrain (11) pendant le mode de mesure au moins pendant une fenêtre de temps ($\Delta t$, $\Delta t_2$) dans laquelle le modèle de corrélation est identifié pour les valeurs mesurées de la deuxième grandeur de mesure (f).

6. Système de mesure selon la revendication 5, pour lequel le premier appareil de terrain (11) et/ou le deuxième appareil de terrain (12) comprend ou comprennent une batterie pour l'alimentation en énergie.

7. Système de mesure selon la revendication 5 ou 6, pour lequel le premier appareil de terrain (11) et/ou le deuxième appareil de terrain (12) est ou sont relié(s) à l'unité de commande (14) au moyen d'une interface sans fil.

**Fig. 1**

Fig. 2

**IN DER BESCHREIBUNG AUFGEFÜHRTE DOKUMENTE**

**In der Beschreibung aufgeführte Nicht-Patentliteratur**

- **TAYEH GABY BOU et al.** *A Spatial-Temporal Correlation Approach for Data Reduction in Cluster-Based Sensor Networks*